# EUROPEAN PATENT SPECIFICATION

(11) **EP 1 159 230 B1**
(45) Date of publication and mention of the grant of the patent: **01.10.2003**
(21) Application number: 00905176.4
(22) Date of filing: 22.02.2000
(51) Int. Cl.: C03C 10/00, C03C 3/062, C03C 3/078

(54) **GLASS-CERAMIC MATERIAL AND PROCESS FOR THE PRODUCTION THEREOF**
GLASKERAMIK UND VERFAHREN ZU DESSEN HERSTELLUNG
COMPOSITE A MATRICE VITRO-CERAMIQUE ET PROCEDE DE PRODUCTION DE CETTE DERNIERE

(30) Priority: 22.02.1999 GB 9904023
(43) Date of publication of application: 05.12.2001
(73) Proprietor: Abonetics Limited, London E1 4NS (GB); De Puy International Limited, Leeds, West Yorks LS11 8DT (GB)
(72) Inventor: BONFIELD, William, Welwyn Hertfordshire AL6 0AT (GB); BRUMMITT, Kenneth, Barnsley South Yorks S75 6EY (GB); BUCKLAND, Thomas, Forest Hill London SE23 2SP (GB)
(74) Representative: Allard, Susan Joyce
(86) International application number: GB0000618
(87) International publication number: WO00050353

(56) References cited:
- EP-A- 0 626 165
- DE-B- 1 007 231
- US-A- 3 997 352

## Description

The present invention relates to glass-ceramic materials and, in particular, to glass-ceramic materials for biomedical applications, such as finger joint prostheses.

A glass-ceramic may be defined as a ceramic which is processed into its final shape as a glass, and is then induced to crystallize by a controlled heat-treatment. Accordingly, glass-ceramics are polycrystalline materials formed by the controlled devitrification of a parent glass. Glass-ceramics generally have improved mechanical properties over the parent glass and are commonly used in the manufacture of kitchenware.

Enstatite is a naturally-occurring magnesium-silicate ceramic whose chemical formula is MgSiO₃. The main commercial application of synthetic enstatite to date has been in the electrical insulator field. Enstatites exhibit relatively high fracture toughness for ceramic materials, thought to be due to the high aspect ratio of the crystallites effectively raising the energy required per unit length to propagate a crack.

US-4,687,749 relates to the production of glass-ceramic articles in which enstatite constitutes the predominant crystal phase. A problem exists, however, in that the crystallization is not controlled and the precursor glasses therefore exhibit marginal glass stability and frequently crack during the crystallization heat-treatment.

The present invention aims to provide a glass-ceramic material having improved mechanical properties and which is suitable for use in biomedical applications.

Accordingly, in a first aspect the present invention provides a glass-ceramic material having a fracture toughness greater than or equal to 3.6 MNm^{-3/2} and comprising:
(a) a major crystalline phase comprising enstatite (MgSiO₃) or one or more polymorphs thereof;
(b) one or more minor crystalline phases comprising at least one of Mg₂SiO₄, SiO₂, TiO₂, MgO, Na₄Mg₂Si₃O₁₀ and/or Na₂Mg₆F₂ (Si₄O₁₁) ₂; and
(c) a residual glassy phase;
which glass-ceramic is obtainable by heating a parent glass composition comprising in the range of from 20 to 60 wt.% MgO, from 35 to 65 wt.% SiO₂, from 1 to 20 wt.% TiO₂ and from 0 to 15 wt.% Na₂O, preferably 1 to 15 wt.% Na₂O.

The chemical composition of the residual glassy phase is difficult to characterise, but it is possible that it comprises one or more of Na₂O, SiO₂, Na₂O-SiO₂ and Na₂O-SiO₂-TiO₂.

The glass-ceramic is preferably obtainable by heating a parent glass composition comprising in the range of from 20 to 35 wt.% MgO, from 50 to 65 wt.% SiO₂, from 4 to 15 wt.% TiO₂ (preferably from 5 to 15 wt.% TiO₂) and from 1 to 12 wt.% Na₂O. More preferably, the glass-ceramic is obtainable by heating a parent glass composition comprising in the range of from 24 to 30 wt.% MgO, from 54 to 62 wt.% SiO₂ (preferably from 54 to 61 wt.% SiO₂), from 4 to 15 wt.% TiO₂ (preferably from 5 to 15 wt.% TiO₂) and from 1 to 10 wt.% Na₂O.

The weight ratio of MgO to SiO₂ is preferably in the range of from 25:75 to 40:60, more preferably from 32:68 to 38:62.

Preferred TiO₂ and Na₂O levels range from 5 to 10 wt.% and 1 to 6 wt.% Na₂O respectively (preferably from 6 to 8 wt.% TiO₂ and 1 to 5 wt.% Na₂O) based on the total weight of the parent glass composition.

The parent glass composition may also comprise a small amount of MnO, for example from 1 to 2 wt.% MnO, for the purpose of stabilising one or more of the polymorphs of enstatite (protoenstatite) in some systems. Whilst the parent glass composition may include other constituents, the presence of Al₂O₃ and/or at least one of Li₂O, CaO, SrO, La₂O₃, Y₂O₃, K₂O and BaO are not essential to the performance of the final material, in contrast to compositions disclosed in US-4,687,749. Accordingly, in a preferred aspect of the present invention the glass-ceramic material consists essentially of the recited phases (a), (b) and (c), together with unavoidable impurities.

The glass-ceramic according to the present invention has a fracture toughness greater than or equal to 3.6 MNm^{-3/2}, although a fracture toughness greater than or equal to 3.8 MNm^{-3/2}, preferably 4 MNm^{-3/2}, more preferably 4.5 MNm^{-3/2}, still more preferably 5 MNm^{-3/2}, can be achieved.

The glass-ceramic according to the present invention typically has a flexural strength greater than or equal to 300 MPa, more typically greater than or equal to 325 MPa, still more typically greater than or equal to 350 MPa.

The glass-ceramic according to the present invention typically has a Vickers hardness of 600 or more for a 20 kg load.

The glass-ceramic according to the present invention typically has an average grain size in the range of from 5 to 15 µm, more typically from 8 to 12 µm, still more typically approximately 10 µm. The microstructure is characterised by the high aspect ratio of the enstatite crystals.

The degree of crystallinity in the glass-ceramic according to the present invention is typically up to 50% by volume, more preferably from 50 to 70% by volume.

The present invention also provides a bone replacement material which comprises a glass-ceramic as herein described.

The present invention also provides a composition which comprises a glass-ceramic or a synthetic bone material as herein described, together with a pharmaceutically acceptable diluent or carrier.

The glass-ceramic, synthetic bone material or composition as herein described may be used in a bone implant, joint implant, prosthesis, such as a finger joint prosthesis, or a filler.

In a second aspect, the present invention provides a process for the production of a glass-ceramic material, which process comprises:
(i) providing a parent glass composition comprising in the range of from 20 to 60 wt.% MgO, from 35 to 65 wt.% SiO₂, from 1 to 20 wt.% TiO₂ and from 0 to 15 wt.% Na₂O, preferably 1 to 15 wt.% Na₂O;
(ii) heating the parent glass composition at a temperature and for a time sufficient to precipitate (a) a major crystalline phase comprising MgSiO₃ or one or more polymorphs thereof, (b) one or more minor crystalline phases comprising at least one of MgSiO₄, SiO₂, MgO, TiO₂, Na₄Mg₂Si₃O₁₀ and/or Na₂Mg₆F₂(Si₄O₁₁)₂, and (c) a residual glassy phase.

In this process, a glass-ceramic component may be formed by first shaping the parent glass, followed by heating to induce crystallization. The parent glass may be formed into the desired shape by any of the glass forming techniques conventional in the art. For example, casting by pouring the molten material into a suitable mould, blow-moulding, a float process, sintering finely divided parent glass particles, centrifugal casting and injection moulding. A particular feature of the process according to the present invention is that cracking is less frequent during casting compared with the prior art parent glass materials.

The parent glass composition preferably comprises in the range of from 20 to 35 wt.% MgO, from 50 to 65 wt.% SiO₂, from 4 to 15 wt.% TiO₂ (preferably from 5 to 15 wt.% TiO₂) and from 1 to 12 wt.% Na₂O. More preferably, the parent glass composition comprises in the range of from 24 to 30 wt.% MgO, from 54 to 62 wt.% SiO₂ (preferably from 54 to 61 wt.% SiO₂), from 4 to 15 wt.% TiO₂ (preferably from 5 to 15 wt.% TiO₂) and from 1 to 10 wt.% Na₂O.

The weight ratio of MgO to SiO₂ is preferably in the range of from 25:75 to 40:60, more preferably from 32:68 to 38:62.

Preferred TiO₂ and Na₂O levels range from 5 to 10 wt.% and 1 to 6 wt.% Na₂O respectively (preferably from 6 to 8 wt.% TiO₂ and preferably from 1 to 5 wt.% Na₂O) based on the total weight of the parent glass composition.

In order to precipitate the various phases, the parent glass composition is typically heated at a temperature in the range of from 600 to 1300°C, preferably from 600 to 1200°C, more preferably from 1000 to 1200°C. Heating is typically carried out at a rate of up to 15°C/minute, preferably up to 10°C/minute, more preferably up to 7.5°C/minute, still more preferably up to 5°C/minute, still more preferably up to 2°C/minute, still more preferably up to 1°C/minute.

In general, a two-stage heat-treatment will be utilised in which the glass is first annealed, followed by controlled heating to produce a large number of nuclei for crystallization and subsequent crystal growth. The annealing step is also preferably carried out at a heating rate of up to 5°C/minute, more preferably up to 2°C/minute, still more preferably up to 1°C/minute. If desired the material may be cooled to room temperature after the annealing step.

A preferred heat-treatment comprises heating the annealed parent composition from room temperature to the peak crystallization temperature (T_{c}), which typically lies in the range of from 750 to 1200°C. Heating is preferably performed at a controlled heating rate of up to 15°C/minute, more preferably up to 10°C/minute, still more preferably up to 5°C/minute, still more preferably up to 2°C/minute, still more preferably up to 1°C/minute. As the glass is heated to the final temperature a large number of dispersed nuclei are produced. Once the final temperature has been reached, the glass is held at that temperature for a time sufficient to produce the desired size of crystallites, typically for 30 minutes or more, more typically for 2 hours or more, still more typically for 10 hours or more. This is then followed by gentle cooling to room temperature, preferably at a rate of up to 10°C/minute, more preferably up to 5°C/minute, still more preferably up to 2°C/minute, still more preferably up to 1°C/minute. Cooling may be carried out in air.

An alternative heat-treatment involves determining the optimum nucleation temperature (Tₙ) of the given system. Tₙ is the temperature at which the maximum number of nuclei are produced in the shortest time within the glass. Tₙ typically lies in the range of from 650 to 900°C, more typically from 700 to 840°C, still more typically from 700 to 750°C. Once Tₙ has been determined, the annealed sample can be raised to Tₙ at a relatively high heating rate, for example up to 20°C/minute, more preferably up to 15°C/minute, still more preferably up to 10°C/minute and held at the temperature for a time sufficient to achieve the desired number of nuclei. The time will generally be from 1 to 5 hours. The material is then heated to T_{c}, again at a relatively high heating rate, for example up to 15°C/minute, more preferably up to 10°C/minute, still more preferably up to 7.5°C/minute. T_{c} typically lies in the range of from 750 to 1200°C. Once the final temperature has been reached, the glass is held at that temperature for a time sufficient to produce the desired size of crystallites. This will typically be for 30 minutes or more, more typically for 2 hours or more, still more typically for 10 hours or more. This is then followed by gentle cooling to room temperature, preferably at a rate of up to 20°C/minute, more preferably up to 10°C/minute, still more preferably up to 5°C/minute, still more preferably up to 2°C/minute. Cooling may be carried out in air. In this manner the rate at which the sample can be heated to or cooled from the two critical temperatures Tₙ and T_{c} depends only on the thermal shock resistance of the sample. Tₙ and T_{c} may be determined in a conventional manner by, for example, differential thermal analysis (DTA).

The parent glass composition may be formed by heating mixed precursor powders comprising oxides and/or carbonates of sodium, silicon, titanium and magnesium, for example Na₂CO₃, SiO₂, TiO₂ and MgCO₃, at a temperature and for a time sufficient to form the parent glass. Heating of the precursor powders will generally involve the conventional steps of melting and homogenisation and optionally refining (or firing). Advantageously, the mixed precursor powders undergo a two-stage heat-treatment comprising: a first step of heating at a temperature in the range of from 800 to 1200°C, preferably 900 to 1100°C, for a time sufficient to reduce the volume of the precursor powders and to initiate the reaction; followed by a second step of heating at a temperature in the range of from 1450 to 1650°C, for a time sufficient to form the parent glass.

Accordingly, the present invention provides a high fracture toughness (≥ 3.6 MNm^{-3/2}) glass-ceramic, castable to near-net shape, intended for use as a joint replacement material in small, non-major load bearing joints, such as finger joints.

The composition and heat-treatment allow controlled crystallization of the desired phases.

The present invention will now be described further with reference to the following examples and drawings, in which:
Figure 1 shows the distribution of X-ray intensity for the glass-ceramic material produced from the composition according to Example 1;
Figure 2 is a scanning electron micrograph of an etched, polished surface of the glass-ceramic material produced from the composition according to Example 1;
Figure 3 shows the heat treatment schedule for determining the optimum nucleation temperature of the composition according to Example 3;
Figure 4 is a scanning electron micrograph of an etched, polished surface of the glass-ceramic material produced from the composition according to Example 4;
Figure 5 is a scanning electron micrograph of an etched, polished surface of the glass-ceramic material produced from the composition according to Example 5;
Figure 6 is an Energy-Dispersive Spectroscopy (EDS) plot the glass-ceramic material produced from the composition according to Example 5;
Figure 7 is a scanning electron micrograph of an etched, polished surface of the glass-ceramic material produced from the composition according to Example 6;
Figure 8 is a scanning electron micrograph of an etched, polished surface of the glass-ceramic material produced from the composition according to Example 7;
Figure 9 shows the distribution of X-ray intensity for the glass-ceramic material produced from the composition according to Example 7;
Figure 10 is a scanning electron micrograph of an etched, polished surface of the glass-ceramic material produced from the composition according to Example 8;
Figure 11 shows the distribution of X-ray intensity for the glass-ceramic material produced from the composition according to Example 8;
Figure 12 is a scanning electron micrograph of an etched, polished surface of the glass-ceramic material produced from the composition according to Example 9; and
Figure 13 shows the distribution of X-ray intensity for the glass-ceramic material produced from the composition according to Example 9.

### Examples 1 and 2

The initial production step is to mix the precursor powders consisting of Na₂CO₃, SiO₂, TiO₂, and MgCO₃ in the appropriate ratios in a pestle and mortar. The Na₂CO₃ is included to reduce the melting temperature and glass viscosity and the TiO₂ is used as a nucleation catalyst. The design composition in weight percents for Examples 1 and 2 are given in Table 1 below. The mixed batch is then transferred to alumina crucibles where the precursor powder is fired at approximately 1000°C for about 3 hours to reduce the volume and initiate the reaction. The 'presintered' precursor is then transferred to a Platinum-Rhodium crucible in an electric furnace at 1550°C in order to form the parent glass. This is fused for a total of 4 hours, being splat-cooled, pulverised and re-melted once after two hours in order to ensure good mixing.

**Table 1**

| Constituent (wt.%) | MgO | SiO₂ | TiO₂ | Na₂O |
|---|---|---|---|---|
| Example 1 | 25.5 | 54.5 | 10 | 10 |
| Example 2 | 27 | 58 | 10 | 5 |

A hot mould is prepared, comprising a two-way split cylinder with a retaining ring, coated with a release agent (Colloidal) graphite suspension, Ascheson Colloids Ltd) and is heated to 600°C. The hot mould is withdrawn from a second electric furnace prior to being filled with the molten glass. The filled mould is then reintroduced into the second furnace and maintained at approximately 600°C for about 1 hour before being cooled at a rate of approximately 1°C/min or less to room temperature. The resulting cylinder of glass is sliced into disks approximately 3mm thick and crystallized by being heated in a third furnace at approximately 1°C/min to 1000°C, where they are held for 3 hours (Example 1) and 10 hours (Example 2) before being cooled, again at approximately 1°C/min, to room temperature.

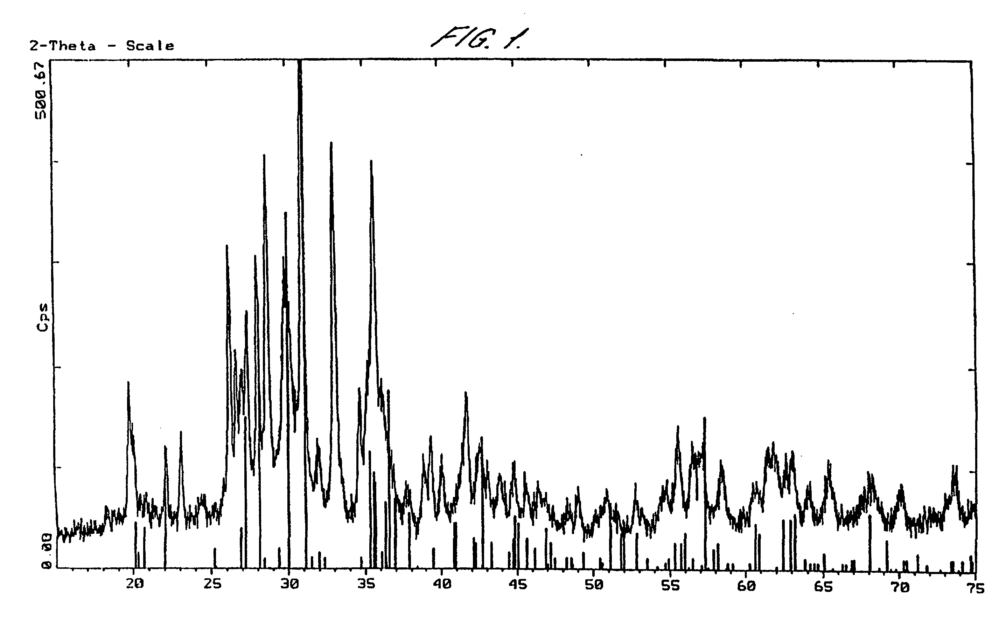

Differential thermal analysis (DTA) has been performed in order to determine the crystallization behaviour, and shows that there are two crystallization events, whose exotherm maximas occur at approximately 840 and 880°C. With reference to Figure 1, X-ray diffraction (XRD) for the glass-ceramic produced from the composition according to Example 1 shows that there are two main phases precipitated, clinoenstatite (MgSiO₃) and crystobalite (SiO₂) . This implies that there is a residual glassy phase consisting of Na₂O-rich silica glass between the crystallites.

Scanning electron microscopy on etched, polished surfaces shows that the microstructure consists of many spherulitic-type grains of enstatite. The fracture surfaces seem to suggest that the fracture path passes around, rather than through, the grains.

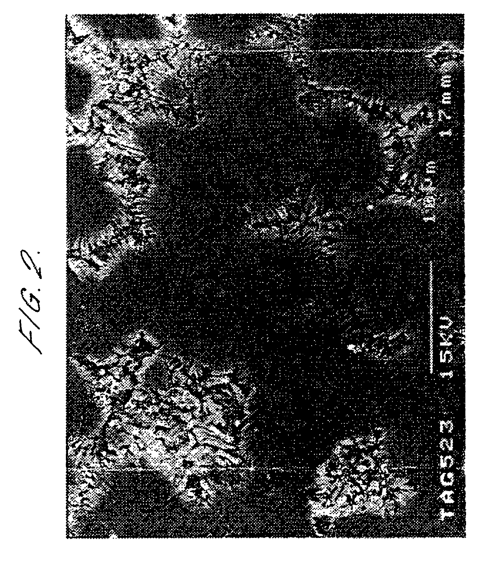

Figure 2 is a SEM micrograph of the final microstructure for the glass-ceramic material produced from the composition according to Example 1. The material was quenched after approximately 90 minutes and etched in 48% HF and the micrograph shows acicular surfaces. The crystallite size is approximately 10 µm.

Flexural testing has been performed on polished samples using the ring-on-ring biaxial flexure test, and shows that the material has a mean flexural strength of 352 MPa with a Weibull modulus of 5.7.

Indentation fracture toughness tests were performed, and showed that the material had a mean fracture toughness of 4.56 MNm^{-3/2} with a standard deviation of 0.79 MNm^{-3/2}, assuming a Young's modulus value of 140 GPa.

MTT assays have shown that the material is non-cytotoxic and behaves similarly to hydroxyapatite. Testing using simulated body fluid (SBF) shows the precipitation of an apatite layer when held at 37°C for 1 week.

### Examples 3 to 9

For Examples 3 to 9, parent glasses were prepared in the same way as in Example 1 above. Weight % and Mole % compositions are shown in Table 2 below. All compositions showed X-ray diffraction patterns consistent with materials containing either proto- or clinoenstatite (MgSiO₃) as major phases, followed by rutile (TiO₂) as a secondary phase and quartz (SiO₂) as a ternary phase when crystallised. There appeared to be a small degree of amorphous scattering indicative of a residual glassy phase in all of the crystallised compositions.

**Table 2**

| Example | Composition / wt% | | | | Composition / mol% | | | |
|---|---|---|---|---|---|---|---|---|
| | MgO | SiO₂ | TiO₂ | Na₂O | MgO | SiO₂ | TiO₂ | Na₂O |
| 3 | 28.5 | 61.2 | 5.00 | 5.28 | 37.7 | 54.4 | 3.34 | 4.55 |
| 4 | 28.8 | 61.9 | 4.00 | 5.33 | 38.0 | 54.8 | 2.66 | 4.57 |
| 5 | 27.9 | 59.9 | 7.00 | 5.17 | 37.2 | 53.6 | 4.71 | 4.48 |
| 6 | 27.0 | 58.0 | 10.0 | 5.00 | 36.4 | 52.4 | 6.80 | 4.38 |
| 7 | 27.9 | 59.9 | 7.00 | 5.17 | 37.2 | 53.6 | 4.71 | 4.48 |
| 8 | 27.9 | 59.9 | 7.00 | 5.17 | 37.2 | 53.6 | 4.71 | 4.48 |
| 9 | 27.9 | 59.9 | 7.00 | 5.17 | 37.2 | 53.6 | 4.71 | 4.48 |

### Example 3

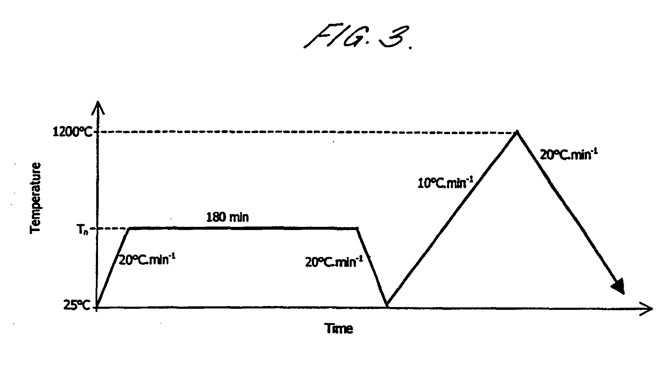

After the parent glass had been produced as described above, small cubes measuring 5 mm to a side were cut using a diamond saw. Six of these cubes were analysed using a differential thermal analyser (Setaram LabSys DTA/DSC 1600) according to the heat treatment shown in Figure 3. The temperature represented by Tₙ in the figure was varied for each sample, being 700, 710, 720, 730, 740 and 750°C. The temperature at which the crystallisation exotherm was maximum was recorded for each sample is given in Table 3 below. A polynomial curve was fitted to a plot of these temperatures on the y-axis against Tₙ on the x-axis. The maximum of this curve, indicative of the optimum nucleation temperature of the composition, was 711°C.

**Table 3**

| **Nucleation hold** | **Peak crystallisation** |
|---|---|
| **temperature (T**_{**n**}**) °C** | **temperature (T**_{**c**}**) °C** |
| 700 | 837 |
| 710 | 844 |
| 720 | 842 |
| 730 | 831 |
| 740 | 824 |
| 750 | 811 |

### Example 4

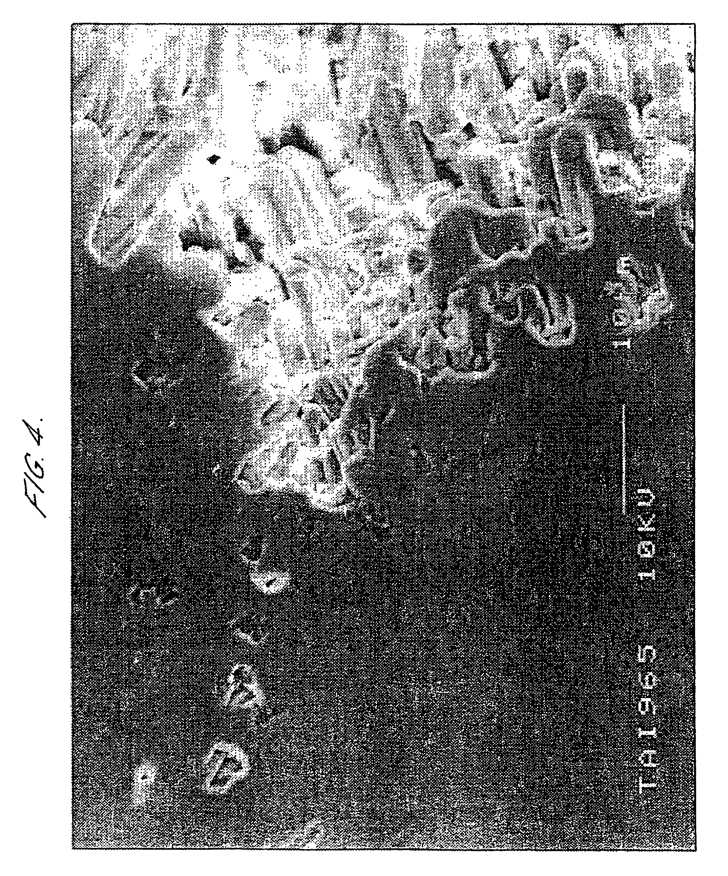

The parent glass was prepared according to the method described previously and the composition described in Table 2. The glass casting was cut into slices approximately 3 mm thick and then crystallised at a heating rate of 10°C/min to 1200°C. The resulting glass-ceramic was then cooled at 10°C/min to room temperature. Specimens were embedded in epoxy resin, and were polished to < 1 mm diamond finish and then examined using scanning electron microscopy (SEM). The structure of the glass ceramic was composed of large, > 1mm in diameter spherulitic crystals with interstitial voids. A SEM micrograph of part of the spherulites and a void is shown in Figure 4.

### Example 5

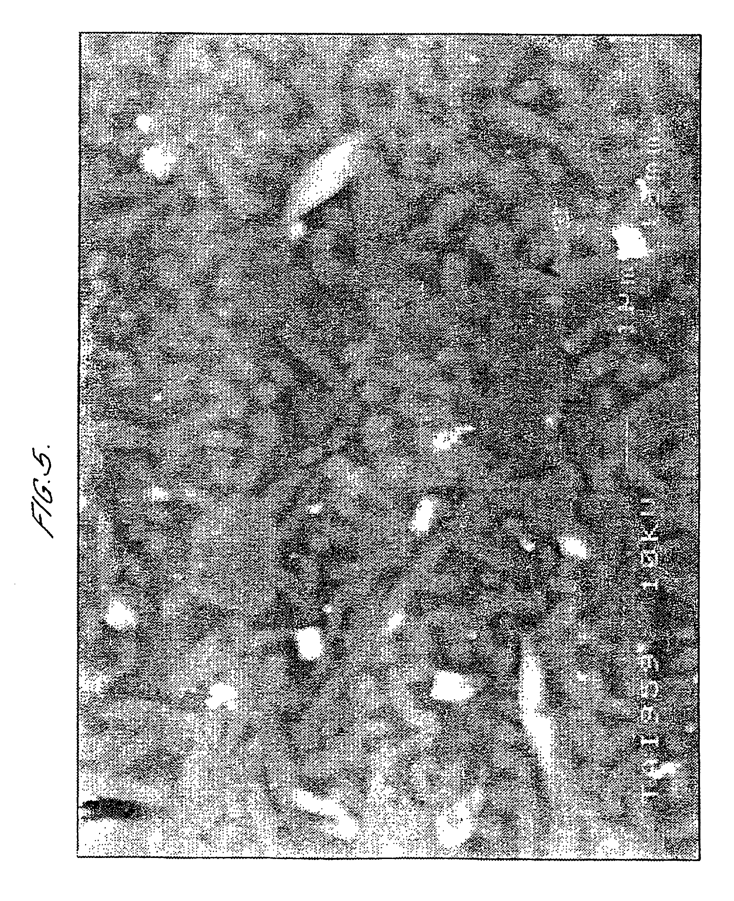

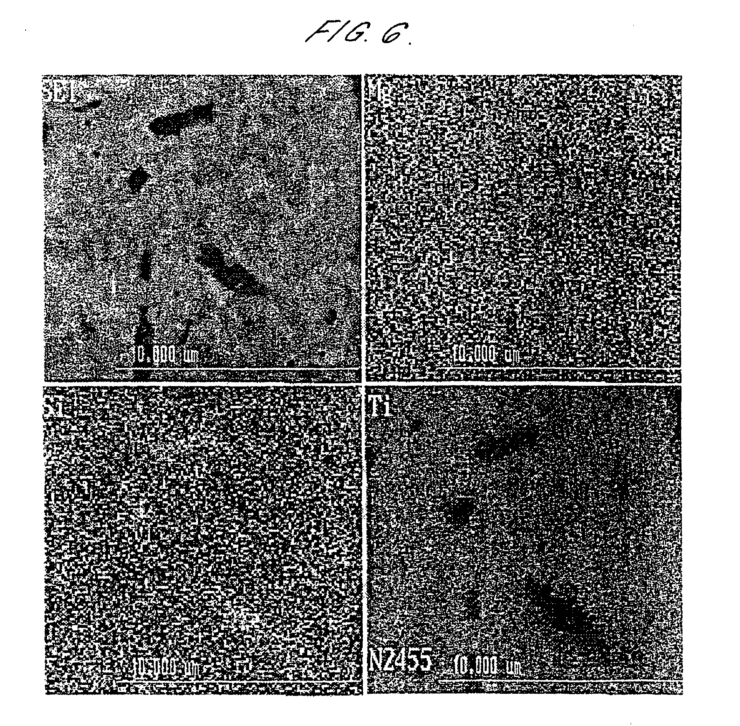

All preparation conditions were the same as for Example 4 apart from the design composition shown in Table 2. SEM showed that the structure now consisted of a uniform dispersion of fine, high aspect ratio crystals varying in their minor dimension from < 0.5 mm to ≈ 1 mm and in major dimension from ≈ 1 to 5 mm (Figure 5). Dispersed throughout the structure were larger, more equally-proportioned grains up to 2 mm in diameter. Energy-dispersive spectroscopy (Figure 6) showed that the white grains also seen in Figure 5 were titanium-rich in contrast to the other grains which were composed of magnesium and silicon, suggesting that the former were the rutile observed in XRD and the latter the enstatite.

### Example 6

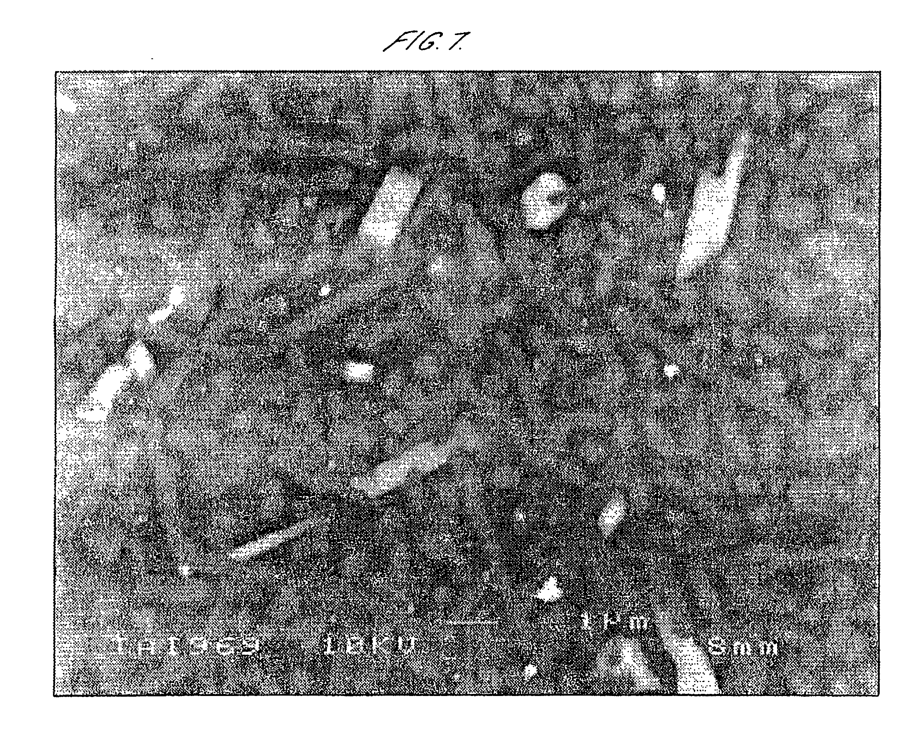

All preparation conditions the same as for Example 5 apart from the design composition shown in Table 2. SEM showed that the structure consisted of the same uniform dispersion of fine, high aspect ratio crystals, but in this case they were generally smaller than those observed in Example 5, and additionally appeared to have lower aspect ratios. There were none of the larger crystallites present, but there appeared to be an increase in the number of rutile crystals (Figure 7).

### Example 7

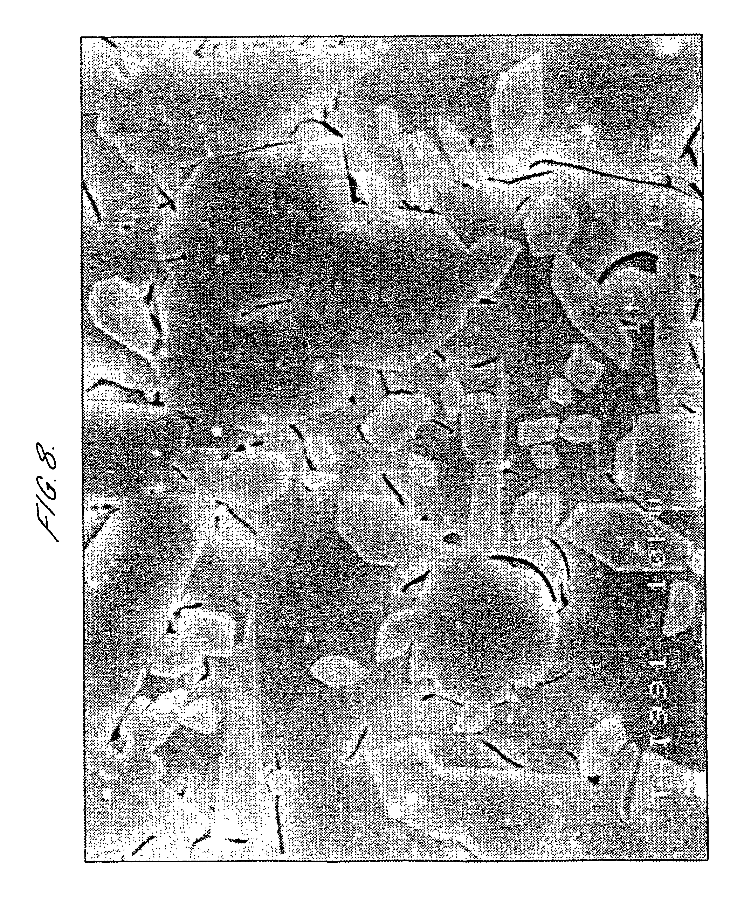

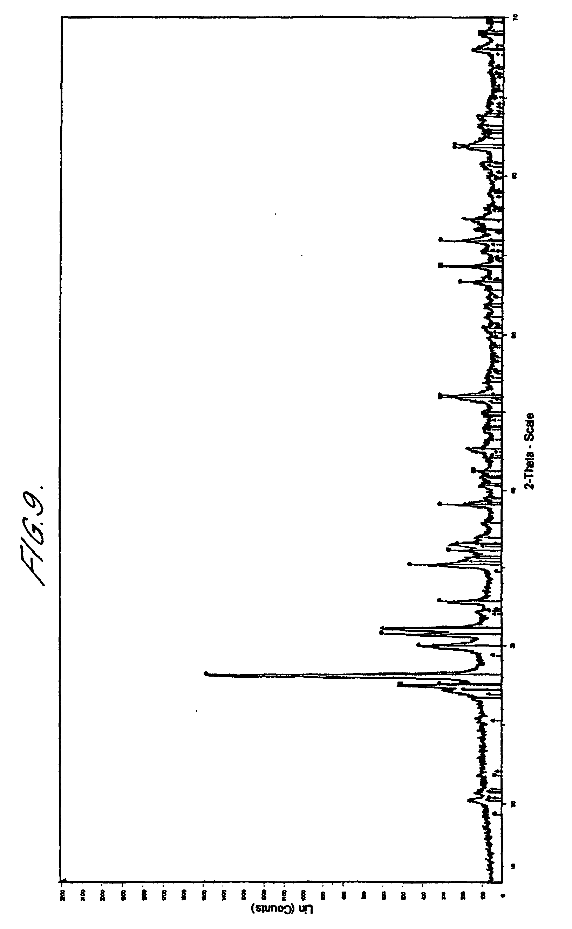

The parent glass was prepared in the same fashion as previously described, but was heated at a rate of 2.5°C/min to a level of 1200°C before being cooled to room temperature at 10°C/min in order to crystallise it. Subsequently, samples were embedded in epoxy resin, sectioned and polished to < 1 mm prior to etching for 15 seconds in a mixture of 1% Hydrofluoric / 5% Hydrochloric acid. The specimens were then gold-coated and examined using SEM. The Example showed that the microstructure consisted of large crystals of enstatite (shown via XRD, Figure 8), frequently > 10 mm in diameter, and that the remaining glassy matrix surrounding them was severely cracked (Figure 9).

### Example 8

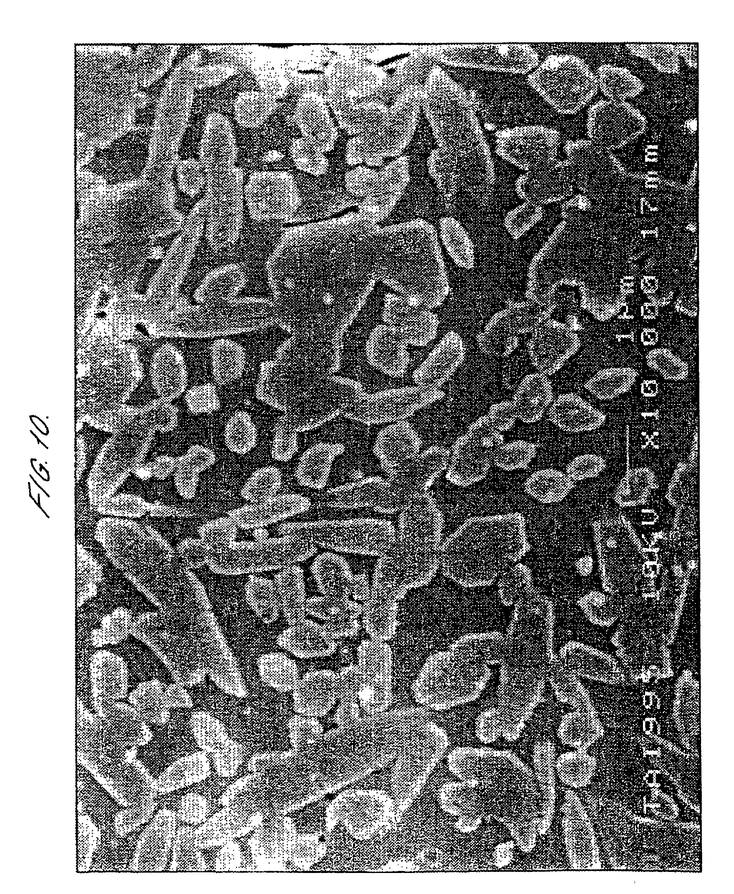

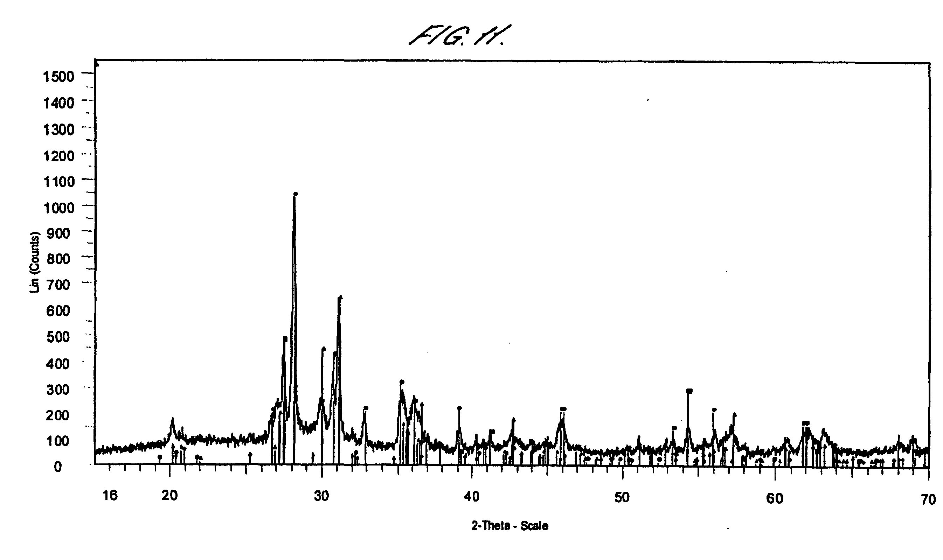

The preparation conditions were the same as before except that the heating rate for crystallisation was 7.5°C/min to 1200°C before cooling. Examination of the crystalline microstructure showed that the enstatite crystals (Figure 10) were much smaller than those in Example 7, and that there was no cracking in the surrounding residual glass matrix. The final degree of crystallinity appeared to be in the order of 50% by volume, and the ratio of proto:clino-enstatite measured via XRD (Figure 11) had decreased in comparison to Example 7.

### Example 9

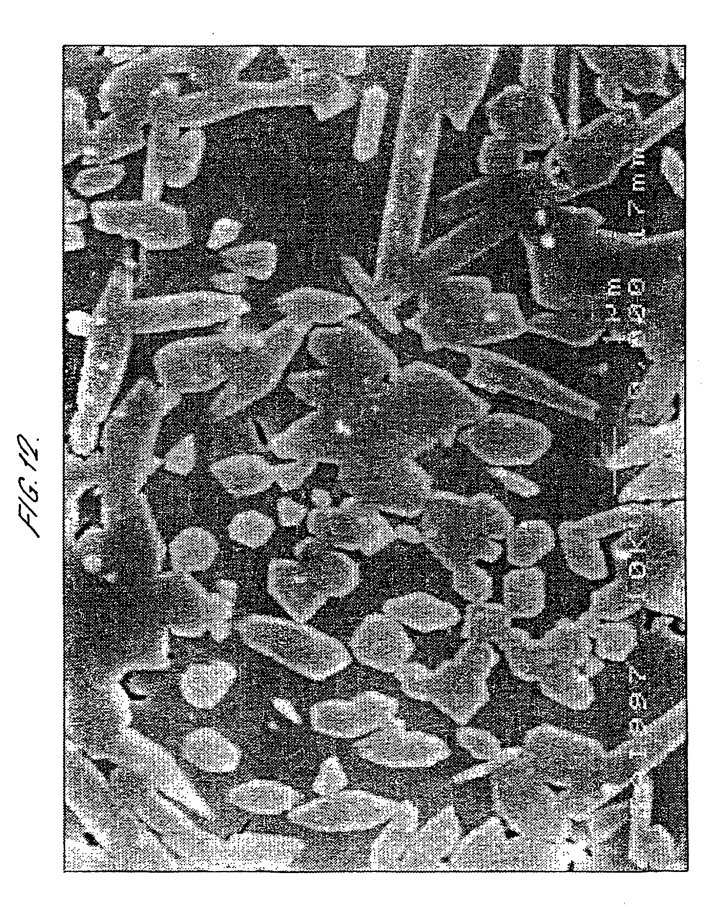

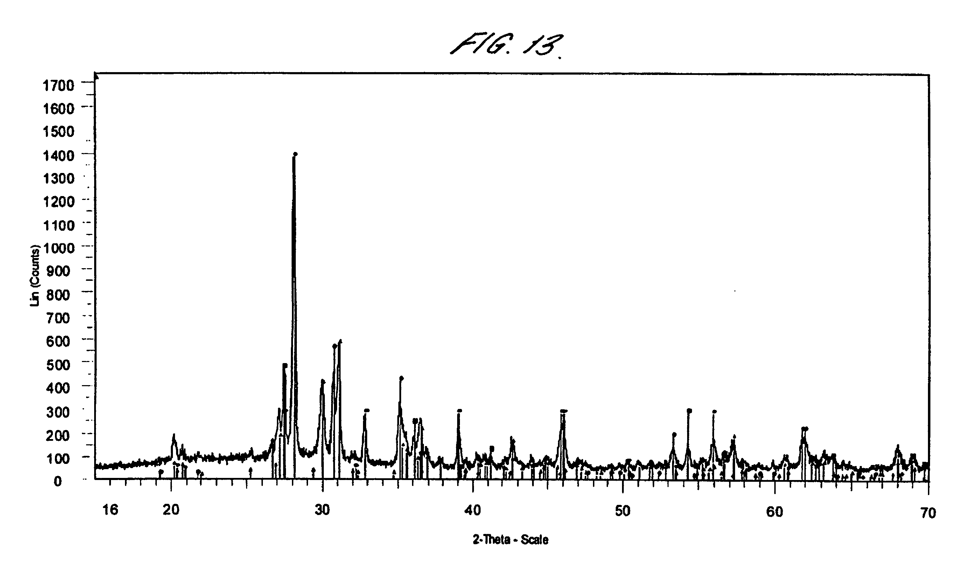

The preparation conditions were the same as before except that the heating rate for crystallisation was 10°C/min to 1200°C before cooling. Again, there was no cracking in the residual glass surrounding the crystalline enstatite phase (Figure 12), but the degree of crystallinity appeared to be lower than that in Example 8. XRD (Figure 13) again showed that the level of protoenstatite had decreased with respect to the level of clinoenstatite.

Indentation fracture toughness tests performed on Examples 3 to 9 showed that the material had a fracture toughness in excess of 3.6 MNm^{-3/2}.

## Claims

1. A glass-ceramic material having a fracture toughness greater than or equal to 3.6 MNm^{-3/2} and comprising:
(a) a major crystalline phase comprising enstatite (MgSiO₃) or one or more polymorphs thereof;
(b) one or more minor crystalline phases comprising at least one of Mg₂SiO₄, SiO₂, TiO₂, MgO, Na₄Mg₂Si₃O₁₀ and/or Na₂Mg₆F₂ (Si₄O₁₁)₂; and
(c) a residual glassy phase;
which glass-ceramic is obtainable by heating a parent glass composition comprising in the range of from 20 to 60 wt.% MgO, from 35 to 65 wt.% SiO₂, from 1 to 20 wt.% TiO₂ and from 1 to 15 wt.% Na₂O.

2. A glass-ceramic as claimed in claim 1 which is obtainable by heating a parent glass composition comprising in the range of from 20 to 35 wt.% MgO, from 50 to 65 wt.% SiO₂, from 4 to 15 wt.% TiO₂ and from 1 to 12 wt.% Na₂O.

3. A glass-ceramic as claimed in claim 1 or claim 2, wherein the weight ratio of MgO to SiO₂ is in the range of from 25:75 to 40:60.

4. A glass-ceramic as claimed in any one of the preceding claims, wherein the weight ratio of MgO to SiO₂ is in the range of from 32:68 to 38:62.

5. A glass-ceramic as claimed in any one of the preceding claims having a fracture toughness greater than or equal to 3.8 MNm^{-3/2}.

6. A glass-ceramic as claimed in claim 5 having a fracture toughness greater than or equal to 4 MNm^{-3/2}.

7. A glass-ceramic as claimed in claim 6 having a fracture toughness greater than or equal to 4.5 MNm^{-3/2}.

8. A glass-ceramic as claimed in claim 7 having a fracture toughness greater than or equal to 5 MNm^{-3/2}.

9. A glass-ceramic as claimed in any one of the preceding claims having a flexural strength greater than or equal to 300 MPa.

10. A glass-ceramic as claimed in claim 9 having a flexural strength greater than or equal to 325 MPa.

11. A glass-ceramic as claimed in claim 10 having a flexural strength greater than or equal to 350 MPa.

12. A glass-ceramic as claimed in any one of the preceding claims having a Vickers hardness (20 kg load) of 600 or more.

13. A glass-ceramic as claimed in any one of the preceding claims, wherein the compositon is free of or substantially free of one or more of Al₂O₃, Li₂O, CaO, SrO, La₂O₃, Y₂O₃, K₂O and BaO.

14. A synthetic bone material which comprises a glass-ceramic as claimed in any one of the preceding claims.

15. A composition which comprises a glass-ceramic as claimed in any one of claims 1 to 13 or a synthetic bone material as claimed in claim 14 together with a pharmaceutically acceptable diluent or carrier.

16. A bone implant, joint implant, prosthesis, filler or cement which comprises a glass-ceramic as claimed in any one of claim 1 to 13, a synthetic bone material as claimed in claim 14 or a composition as claimed in claim 15.

17. A prosthesis as claimed in claim 16 which is a finger joint prosthesis.

18. A glass-ceramic as claimed in any one of claims 1 to 13, a synthetic bone material as claimed in claim 14 or a composition as claimed in claim 15 for use in a method of treatment of the human or animal body by surgery or therapy.

19. A process for the production of a glass-ceramic material, which process comprises:
(i) providing a parent glass composition comprising in the range of from 20 to 60 wt.% MgO, from 35 to 65 wt.% SiO₂, from 1 to 20 wt.% TiO₂ and from 1 to 15 wt.% Na₂O;
(ii) heating the parent glass composition at a temperature and for a time sufficient to precipitate (a) a major crystalline phase comprising MgSiO₃ or one or more polymorphs thereof, (b) one or more minor crystalline phases comprising at least one of Mg₂SiO₄, SiO₂, MgO, TiO₂, Na₄Mg₂Si₃O₁₀ and/or Na₂Mg₆F₂(Si₄O₁₁)₂, and (c) a residual glassy phase.

20. A process as claimed in claim 19, wherein the parent glass composition comprises in the range of from 20 to 35 wt.% MgO, from 50 to 65 wt.% SiO₂, from 4 to 15 wt.% TiO₂ and from 1 to 12 wt.% Na₂O.

21. A process as claimed in claim 19 or claim 20, wherein the parent glass composition comprises in the range of from 24 to 30 wt.% MgO, from 54 to 62 wt.% SiO₂, from 5 to 10 wt.% TiO₂ and from 1 to 10 wt.% Na₂O.

22. A process as claimed in any one of claims 19 to 21, wherein the parent glass composition is heated to a temperature in the range of from 750 to 1200°C to precipitate the said phases.

23. A process as claimed in claim 22, wherein the parent glass composition is heated to a temperature in the range of from 800 to 1100°C to precipitate the said phases.

24. A process as claimed in any one of claims 19 to 23, wherein the parent glass composition is heated at a heating rate of up to 20°C/minute, preferably up to 15°C/minute, more preferably up to 10°C/minute, still more preferably up to 5°C/minute.

25. A process as claimed in any one of claims 19 to 24, wherein the parent glass composition is cooled at a cooling rate of up to 20°C/minute, preferably up to 15°C/minute, more preferably up to 10°C/minute, still more preferably up to 5°C/minute following the step of heating to precipitate the said phases.

26. A process as claimed in any one of claims 19 to 21, wherein the step of heating the parent glass composition comprises the following steps:
(a) heating to Tₙ at a rate of up to 20°C/minute, preferably up to 15°C/minute, more preferably up to 10°C/minute, wherein Tₙ is the temperature at which the maximum number of nuclei are produced in the shortest time within the parent glass composition;
(b) maintaining the composition at Tₙ for a time until the desired number of nuclei have been achieved;
(c) heating to T_{c} at a rate of up 15°C/minute, more preferably up to 10°C/minute, still more preferably up to 7.5°C/minute, wherein T_{c} is the peak crystallization temperature;
(d) maintaining the composition at T_{c} for a time until the desired crystallite size has been achieved; and
(e) cooling the resulting glass-ceramic material.

27. A process as claimed in claim 26, wherein Tₙ lies in the range of from 700 to 840°C, preferably 700 to 750°C, more preferably 700 to 730°C, still more preferably 705 to 715°C.

28. A process as claimed in claim 26 or claim 27, wherein T_{c} lies in the range of from 750 to 1200°C.

29. A process as claimed any one of claim 26 to 28, wherein heating to T_{c} is carried out at a rate of from 2.5 to 10°C/minute, preferably from 5 to 10°C/minute, more preferably from 7.5 to 10°C/minute

30. A process as claimed in any one of claims 19 to 29, wherein the parent glass composition is annealed prior to the step of heating to precipitate the said phases.

31. A process as claimed in any one of claims 19 to 30, wherein the parent glass composition is formed by heating mixed precursor powders comprising Na₂CO₃, SiO₂, TiO₂ and MgCO₃ at a temperature and for a time sufficient to form the parent glass.

32. A process as claimed in claim 31, wherein the mixed precursor powders undergo a heat-treatment comprising heating at a temperature in the range of from 1450 to 1650°C for a time sufficient to form the parent glass.

33. A process as claimed in claim 31, wherein the mixed precursor powders undergo a two-stage heat-treatment comprising a first step of heating at a temperature in the range of from 800 to 1200°C, followed by a second stage of heating at a temperature in the range of from 1450 to 1650°C.

34. A process as claimed in any one of claims 19 to 33, wherein molten parent glass is cast into a desired shape prior to the steps of heating to precipitate the various phases.

35. A process as claimed in any one of claims 19 to 34, wherein the parent glass is free of or substantially free of one or more of Al₂O₃, Li₂O, CaO, SrO, La₂O₃, Y₂O₃, K₂O and BaO.

36. A process as claimed in any one of claims 19 to 35, wherein the parent glass consists essentially of MgO, SiO₂, TiO₂ and Na₂O, together with unavoidable impurities.

## Patentansprüche

1. Glaskeramik-Material, das eine Bruchzähigkeit von größer als oder gleich 3,6 MNm^{-3/2} aufweist und umfasst:
(a) eine kristalline Hauptphase, umfassend Enstatit (MgSiO₃) oder eine oder mehrere polymorphe Formen davon;
(b) eine oder mehrere kristalline Nebenphasen, umfassend mindestens eine von Mg₂SiO₄, SiO₂, TiO₂, MgO, Na₄Mg₂Si₃O₁₀ und/oder Na₂Mg₆F₂(Si₄O₁₁)₂; und
(c) eine restliche glasartige Phase;
wobei die Glaskeramik erhältlich ist, indem eine Ausgangsglaszusammensetzung, die im Bereich von 20 bis 60 Gew.-% MgO, von 35 bis 65 Gew.-% SiO₂, von 1 bis 20 Gew.-% TiO₂ und von 1 bis 15 Gew.-% Na₂O umfasst, erhitzt wird.

2. Glaskeramik nach Anspruch 1, die erhältlich ist, indem eine Ausgangsglaszusammensetzung, die im Bereich von 20 bis 35 Gew.-% MgO, von 50 bis 65 Gew.-% SiO₂, von 4 bis 15 Gew.-% TiO₂ und von 1 bis 12 Gew.-% Na₂O umfasst, erhitzt wird.

3. Glaskeramik nach Anspruch 1 oder Anspruch 2, wobei das Gewichtsverhältnis von MgO zu SiO₂ im Bereich von 25:75 bis 40:60 liegt.

4. Glaskeramik nach einem der vorhergehenden Ansprüche, wobei das Gewichtsverhältnis von MgO zu SiO₂ im Bereich von 32:68 bis 38:62 liegt.

5. Glaskeramik nach einem der vorhergehenden Ansprüche, die eine Bruchzähigkeit von größer als oder gleich 3,8 MNm^{-3/2} aufweist.

6. Glaskeramik nach Anspruch 5, die eine Bruchzähigkeit von größer als oder gleich 4 MNm^{-3/2} aufweist.

7. Glaskeramik nach Anspruch 6, die eine Bruchzähigkeit von größer als oder gleich 4,5 MNm^{-3/2} aufweist.

8. Glaskeramik nach Anspruch 7, die eine Bruchzähigkeit von größer als oder gleich 5 MNm^{-3/2} aufweist.

9. Glaskeramik nach einem der vorhergehenden Ansprüche, die eine Biegefestigkeit von größer als oder gleich 300 MPa aufweist.

10. Glaskeramik nach Anspruch 9, die eine Biegefestigkeit von größer als oder gleich 325 MPa aufweist.

11. Glaskeramik nach Anspruch 10, die eine Biegefestigkeit von größer als oder gleich 350 MPa aufweist.

12. Glaskeramik nach einem der vorhergehenden Ansprüche, die eine Vickershärte (20 kg Last) von 600 oder mehr aufweist.

13. Glaskeramik nach einem der vorhergehenden Ansprüche, wobei die Zusammensetzung frei oder im Wesentlichen frei ist von einer oder mehreren von Al₂O₃, Li₂O, CaO, SrO, La₂O₃, Y₂O₃, K₂O und BaO.

14. Synthetisches Knochenmaterial, das eine Glaskeramik nach einem der vorhergehenden Ansprüche umfasst.

15. Zusammensetzung, die eine Glaskeramik nach einem der Ansprüche 1 bis 13 oder ein synthetisches Knochenmaterial nach Anspruch 14 zusammen mit einem pharmazeutisch annehmbaren Verdünnungsmittel oder Träger umfasst.

16. Knochenimplantat, Gelenkimplantat, Prothese, Füllmittel oder Zement, umfassend eine Glaskeramik nach einem der Ansprüche 1 bis 13, ein synthetisches Knochenmaterial nach Anspruch 14 oder eine Zusammensetzung nach Anspruch 15.

17. Prothese nach Anspruch 16, die eine Fingergelenkprothese ist.

18. Glaskeramik nach einem der Ansprüche 1 bis 13, synthetisches Knochenmaterial nach Anspruch 14 oder Zusammensetzung nach Anspruch 15 zur Verwendung in einem Verfahren zur Behandlung des menschlichen oder tierischen Körpers durch Operation oder Therapie.

19. Verfahren zur Herstellung eines Glaskeramik-Materials, wobei das Verfahren umfasst:
(i) Bereitstellen einer Ausgangsglaszusammensetzung, umfassend im Bereich von 20 bis 60 Gew.-% MgO, von 35 bis 65 Gew.-% SiO₂, von 1 bis 20 Gew.-% TiO₂ und von 1 bis 15 Gew.-% Na₂O;
(ii) Erhitzen der Ausgangsglaszusammensetzung bei einer Temperatur und für eine Zeitdauer, die ausreichen, um (a) eine kristalline Hauptphase, umfassend MgSiO₃ oder eine oder mehrere polymorphe Formen davon, (b) eine oder mehrere kristalline Nebenphasen, umfassend mindestens eine von Mg₂SiO₄, SiO₂, MgO, TiO₂ Na₄Mg₂Si₃O₁₀ und/oder Na₂Mg₆F₂(Si₄O₁₁)₂, und (c) eine restliche glasartige Phase abzuscheiden.

20. Verfahren nach Anspruch 19, wobei die Ausgangsglaszusammensetzung im Bereich von 20 bis 35 Gew.-% MgO, von 50 bis 65 Gew.-% SiO₂, von 4 bis 15 Gew.-% TiO₂ und von 1 bis 12 Gew.-% Na₂O umfasst.

21. Verfahren nach Anspruch 19 oder Anspruch 20, wobei die Ausgangsglaszusammensetzung im Bereich von 24 bis 30 Gew.-% MgO, von 54 bis 62 Gew.-% SiO₂, von 5 bis 10 Gew.-% TiO₂ und von 1 bis 10 Gew.-% Na₂O umfasst.

22. Verfahren nach einem der Ansprüche 19 bis 21, wobei die Ausgangsglaszusammensetzung auf eine Temperatur im Bereich von 750 bis 1200°C erhitzt wird, um die Phasen abzuscheiden.

23. Verfahren nach Anspruch 22, wobei die Ausgangsglaszusammensetzung auf eine Temperatur im Bereich von 800 bis 1100°C erhitzt wird, um die Phasen abzuscheiden.

24. Verfahren nach einem der Ansprüche 19 bis 23, wobei die Ausgangsglaszusammensetzung mit einer Aufheizgeschwindigkeit von bis zu 20°C/Minute, vorzugsweise bis zu 15°C/Minute, mehr bevorzugt bis zu 10°C/Minute, noch mehr bevorzugt bis zu 5°C/Minute erhitzt wird.

25. Verfahren nach einem der Ansprüche 19 bis 24, wobei die Ausgangsglaszusammensetzung nach dem Schritt des Erhitzens zur Abscheidung der Phasen mit einer Kühlgeschwindigkeit von bis zu 20°C/Minute, vorzugsweise bis zu 15°C/Minute, mehr bevorzugt bis zu 10°C/Minute, noch mehr bevorzugt bis zu 5°C/Minute gekühlt wird.

26. Verfahren nach einem der Ansprüche 19 bis 21, wobei der Schritt des Erhitzens der Ausgangsglaszusammensetzung die folgenden Schritte umfasst:
(a) Erhitzen auf Tₙ mit einer Geschwindigkeit von bis zu 20°C/Minute, vorzugsweise bis zu 15°C/Minute, mehr bevorzugt bis zu 10°C/Minute, wobei Tₙ die Temperatur ist, bei der in der kürzesten Zeitdauer die maximale Menge an Kristallisationskernen innerhalb der Ausgangsglaszusammensetzung erzeugt werden;
(b) Halten der Zusammensetzung bei Tₙ für eine Zeitdauer, bis die gewünschte Menge an Kristallisationskernen erhalten wurde;
(c) Erhitzen auf T_{c} mit einer Geschwindigkeit von bis zu 15°C/Minute, mehr bevorzugt bis zu 10°C/Minute, noch mehr bevorzugt bis zu 7,5°C/Minute, wobei T_{c} die Kristallisationspeaktemperatur ist;
(d) Halten der Zusammensetzung bei T_{c} für eine Zeitdauer, bis die gewünschte Kristallitgröße erhalten wurde; und
(e) Kühlen des resultierenden Glaskeramik-Materials.

27. Verfahren nach Anspruch 26, wobei Tₙ im Bereich von 700 bis 840°C, vorzugsweise 700 bis 750°C, mehr bevorzugt 700 bis 730°C, noch mehr bevorzugt 705 bis 715°C liegt.

28. Verfahren nach Anspruch 26 oder Anspruch 27, wobei T_{c} im Bereich von 750 bis 1200°C liegt.

29. Verfahren nach einem der Ansprüche 26 bis 28, wobei das Erhitzen auf T_{c} mit einer Geschwindigkeit von 2,5 bis 10°C/Minute, vorzugsweise von 5 bis 10°C/Minute, mehr bevorzugt von 7,5 bis 10°C/Minute durchgeführt wird.

30. Verfahren nach einem der Ansprüche 19 bis 29, wobei die Ausgangsglaszusammensetzung vor dem Schritt des Erhitzens zur Abscheidung der Phasen getempert wird.

31. Verfahren nach einem der Ansprüche 19 bis 30, wobei die Ausgangsglaszusammensetzung gebildet wird, indem gemischte Vorläufer-Pulver umfassend Na₂CO₃, SiO₂, TiO₂ und MgCO₃ bei einer Temperatur und für eine Zeitdauer erhitzt werden, die ausreichen, damit das Ausgangsglas gebildet wird.

32. Verfahren nach Anspruch 31, wobei die gemischten Vorläufer-Pulver eine Hitzebehandlung durchlaufen, die das Erhitzen bei einer Temperatur im Bereich von 1450 bis 1650°C für eine Zeitdauer umfasst, die ausreicht, damit das Ausgangsglas gebildet wird.

33. Verfahren nach Anspruch 31, wobei die gemischten Vorläufer-Pulver eine zweistufige Hitzebehandlung durchlaufen, die einen ersten Schritt des Erhitzens bei einer Temperatur im Bereich von 800 bis 1200°C, gefolgt von einer zweiten Stufe des Erhitzens bei einer Temperatur im Bereich von 1450 bis 1650°C umfasst.

34. Verfahren nach einem der Ansprüche 19 bis 33, wobei geschmolzenes Ausgangsglas vor den Schritten des Erhitzens zur Abscheidung der verschiedenen Phasen in eine gewünschte Form gegossen wird.

35. Verfahren nach einem der Ansprüche 19 bis 34, wobei das Ausgangsglas frei oder im Wesentlichen frei ist von einer oder mehreren von Al₂O₃, Li₂O, CaO, SrO, La₂O₃, Y₂O₃, K₂O und BaO.

36. Verfahren nach einem der Ansprüche 19 bis 35, wobei das Ausgangsglas im Wesentlichen aus MgO, SiO₂, TiO₂ und Na₂O, zusammen mit unvermeidbaren Verunreinigungen, besteht.

## Revendications

1. Matériau vitrocéramique ayant une ténacité à la rupture supérieure ou égale à 3,6 MNm^{-3/2} et comprenant :
(a) une phase cristalline dominante comprenant de l'enstatite (MgSiO₃) ou bien une ou plusieurs de ses formes polymorphes ;
(b) une ou plusieurs phases cristallines secondaires comprenant au moins une des entités consistant en Mg₂SiO₄, SiO₂, TiO₂, MgO, Na₄Mg₂Si₃O₁₀ et/ou Na₂Mg₆F₂ (Si₄O₁₁)₂ ; et
(c) une phase vitreuse résiduelle ;
matériau vitrocéramique qui peut être obtenu en chauffant une composition de verre de départ comprenant 20 à 60% en poids de MgO, 35 à 65% en poids de SiO₂, 1 à 20% en poids de TiO₂ et 1 à 15% en poids de Na₂O.

2. Matériau vitrocéramique suivant la revendication 1, qui peut être obtenu en chauffant une composition de verre de départ comprenant 20 à 35% en poids de MgO, 50 à 65% en poids de SiO₂, 4 à 15% en poids de TiO₂ et 1 à 12% en poids de Na₂O.

3. Matériau vitrocéramique suivant la revendication 1 ou la revendication 2, dans lequel le rapport pondéral de MgO à SiO₂ est compris dans l'intervalle de 25:75 à 40:60.

4. Matériau vitrocéramique suivant l'une quelconque des revendications précédentes, dans lequel le rapport pondéral de MgO à SiO₂ est compris dans l'intervalle de 32:68 à 38:62.

5. Matériau vitrocéramique suivant l'une quelconque des revendications précédentes, ayant une ténacité à la rupture supérieure ou égale à 3,8 MNm^{-3/2}.

6. Matériau vitrocéramique suivant la revendication 5, ayant une ténacité à la rupture supérieure ou égale à 4 MNm^{-3/2}.

7. Matériau vitrocéramique suivant la revendication 6, ayant une ténacité à la rupture supérieure ou égale à 4 , 5 MNm^{-3/2} .

8. Matériau vitrocéramique suivant la revendication 7, ayant une ténacité à la rupture supérieure ou égale à 5 MNm^{-3/2}.

9. Matériau vitrocéramique suivant l'une quelconque des revendications précédentes, ayant une résistance à la flexion supérieure ou égale à 300 MPa.

10. Matériau vitrocéramique suivant la revendication 9, ayant une résistance à la flexion supérieure ou égale à 325 MPa.

11. Matériau vitrocéramique suivant la revendication 10, ayant une résistance à la flexion supérieure ou égale à 350 MPa.

12. Matériau vitrocéramique suivant l'une quelconque des revendications précédentes, ayant une dureté Vickers (charge de 20 kg) égale ou supérieure à 600.

13. Matériau vitrocéramique suivant l'une quelconque des revendications précédentes, dans lequel la composition est dépourvue ou pratiquement dépourvue d'une ou plusieurs des entités consistant en Al₂O₃, Li₂O, CaO, SrO, La₂O₃, Y₂O₃, K₂O et BaO.

14. Matériau osseux synthétique qui comprend un matériau vitrocéramique suivant l'une quelconque des revendications précédentes.

15. Composition qui comprend un matériau vitrocéramique suivant l'une quelconque des revendications 1 à 13 ou un matériau osseux synthétique suivant la revendication 14 conjointement avec un diluant ou support pharmaceutiquement acceptable.

16. Implant osseux, implant articulaire, prothèse, charge ou ciment qui comprend un matériau vitrocéramique suivant l'une quelconque des revendications 1 à 13, un matériau osseux synthétique suivant la revendication 14 ou une composition suivant la revendication 15.

17. Prothèse suivant la revendication 16, qui est une prothèse d'articulation du doigt.

18. Matériau vitrocéramique suivant l'une quelconque des revendications 1 à 13, matériau osseux synthétique suivant la revendication 14 ou composition suivant la revendication 15, destiné à être utilisé dans une méthode de traitement du corps de l'homme et d'un animal par chirurgie ou thérapie.

19. Procédé pour la production d'un matériau vitrocéramique, procédé qui comprend les étapes consistant :
(i) à fournir une composition de verre de départ comprenant 20 à 60% en poids de MgO, 35 à 65% en poids de SiO₂, 1 à 20% en poids de TiO₂, et 1 à 15% en poids de Na₂O ;
(ii) à chauffer la composition de verre de départ à une température et pendant un temps suffisants pour précipiter (a) une phase cristalline dominante comprenant du MgSiO₃ ou bien une ou plusieurs de ses formes polymorphes, (b) une ou plusieurs phases cristallines secondaires comprenant au moins une des entités consistant en Mg₂SiO₄, SiO₂, MgO, TiO₂, Na₄Mg₂Si₃O₁₀ et/ou Na₂Mg₆F₂(Si₄O₁₁)₂, et (c) une phase vitreuse résiduelle.

20. Procédé suivant la revendication 19, dans lequel la composition de verre de départ comprend 20 à 35% en poids de MgO, 50 à 65% en poids de SiO₂, 4 à 15% en poids de TiO₂ et 1 à 12% en poids de Na₂O.

21. Procédé suivant la revendication 19 ou la revendication 20, dans lequel la composition de verre de départ comprend 24 à 30% en poids de MgO, 54 à 62% en poids de SiO₂, 5 à 10% en poids de TiO₂ et 1 à 10% en poids de Na₂O.

22. Procédé suivant l'une quelconque des revendications 19 à 21, dans lequel la composition de verre de départ est chauffée à une température comprise dans l'intervalle de 750 à 1200°C pour précipiter lesdites phases solides.

23. Procédé suivant la revendication 22, dans lequel la composition de verre de départ est chauffée à une température comprise dans l'intervalle de 800 à 1100°C pour précipiter lesdites phases.

24. Procédé suivant l'une quelconque des revendications 19 à 23, dans lequel la composition de verre de départ est chauffée à une vitesse d'élévation de température allant jusqu'à 20°C/min, avantageusement jusqu'à 15°C/min, plus avantageusement jusqu'à 10°C/min, de préférence jusqu'à 5°C/min.

25. Procédé suivant l'une quelconque des revendications 19 à 24, dans lequel la composition de verre de départ est refroidie à une vitesse de refroidissement allant jusqu'à 20°C/min, avantageusement jusqu'à 15°C/min, plus avantageusement jusqu'à 10°C/min, de préférence jusqu'à 5°C/min après l'étape de chauffage pour précipiter lesdites phases.

26. Procédé suivant l'une quelconque des revendications 19 à 21 dans lequel l'étape de chauffage de la composition de verre de départ comprend les étapes suivantes :
(a) chauffage à Tₙ à une vitesse allant jusqu'à 20°C/min, avantageusement jusqu'à 15°C/min, plus avantageusement jusqu'à 10°C/min, Tₙ étant la température à laquelle le nombre maximal de germes cristallins est produit dans le temps le plus bref dans la composition des verres de départ ;
(b) maintien de la composition à Tₙ pendant un temps jusqu'à ce que le nombre désiré de germes cristallins ait été obtenu ;
(c) chauffage à T_{c} à une vitesse allant jusqu'à 15°C/min, plus avantageusement jusqu'à 10°C/min, de préférence jusqu'à 7,5°C/min, T_{c} étant la température de cristallisation maximale ;
(d) maintien de la composition à T_{c} pendant un temps jusqu'à ce que les dimensions désirées des cristallites ait été obtenues ; et
(e) refroidissement du matériau vitrocéramique résultant.

27. Procédé suivant la revendication 26, dans lequel Tₙ est comprise dans l'intervalle de 700 à 840°C, avantageusement de 700 à 750°C, plus avantageusement de 700 à 730°C, de préférence de 705 à 715°C.

28. Procédé suivant la revendication 26 ou la revendication 27, dans lequel T_{c} est comprise dans l'intervalle de 750 à 1200°C.

29. Procédé suivant l'une quelconque des revendications 26 à 28, dans lequel le chauffage à T_{c} est effectué à une vitesse de 2,5 à 10°C/min, avantageusement de 5 à 10°C/min, plus avantageusement de 7,5 à 10°C .

30. Procédé suivant l'une quelconque des revendications 19 à 29, dans lequel la composition de verre de départ est soumise à un recuit avant l'étape de chauffage pour précipiter lesdites phases.

31. Procédé suivant l'une quelconque des revendications 19 à 30, dans lequel la composition de verre de départ est formée en chauffant des poudres de précurseurs mixtes comprenant Na₂CO₃, SiO₂, TiO₂ et MgCO₃ à une température et pendant un temps suffisants pour former le verre de départ.

32. Procédé suivant la revendication 31, dans lequel les poudres de précurseurs mixtes subissent un traitement thermique comprenant un chauffage à une température comprise dans l'intervalle de 1450 à 1650°C pendant un temps suffisant pour former le verre de départ.

33. Procédé suivant la revendication 31, dans lequel les poudres de précurseurs mixtes subissent un traitement thermique en deux étapes, comprenant une première étape de chauffage à une température comprise dans l'intervalle de 800 à 1200°C, suivie par une seconde étape de chauffage à une température comprise dans l'intervalle de 1450 à 1650°C.

34. Procédé suivant l'une quelconque des revendications 19 à 33, dans lequel le verre de départ fondu est coulé sous une forme désirée avant les étapes de chauffage pour précipiter les diverses phases.

35. Procédé suivant l'une quelconque des revendications 19 à 34, dans lequel le verre de départ est dépourvu ou pratiquement dépourvu d'une ou plusieurs des entités consistant en Al₂O₃, Li₂O, CaO, SrO, La₂O₃, Y₂O₃, K₂O et BaO.

36. Procédé suivant l'une quelconque des revendications 19 à 35, dans lequel le verre de départ consiste essentiellement en MgO, SiO₂, TiO₂ et Na₂O, conjointement avec les impuretés inévitables.
